# EUROPÄISCHE PATENTSCHRIFT

(11) **EP 3 177 338 B1**
(45) Veröffentlichungstag und Bekanntmachung des Hinweises auf die Patenterteilung: **15.12.2021**
(21) Anmeldenummer: 15748179.7
(22) Anmeldetag: 05.08.2015
(51) Int. Cl.: A61M 1/36

(54) **VERFAHREN ZUM AUSWASCHEN VON GASBLASEN IN EINEM EXTRAKORPORALEN BLUTKREISLAUF**
METHOD FOR WASHING OUT GAS BUBBLES IN AN EXTRACORPOREAL BLOOD CIRCUIT
PROCÉDÉ D'ÉLIMINATION PAR LAVAGE DE BULLES DE GAZ D'AU MOINS UNE ZONE CIBLE D'UN CIRCUIT DE SANG EXTRACORPOREL

(30) Priorität: 05.08.2014 DE 102014011675
(43) Veröffentlichungstag der Anmeldung: 14.06.2017
(73) Patentinhaber: Fresenius Medical Care Deutschland GmbH, 61352 Bad Homburg (DE)
(72) Erfinder: WOJKE, Ralf, 61348 Bad Homburg (DE); WIENEKE, Paul, 48149 Münster (DE)
(74) Vertreter: Herrmann, Uwe
(86) Internationale Anmeldenummer: PCT/EP2015/001618
(87) Internationale Veröffentlichungsnummer: WO 2016/020061

(56) Entgegenhaltungen:
- EP-A1- 0 834 329
- EP-A1- 1 892 000
- EP-A1- 2 462 965
- EP-A2- 0 543 172
- DE-A1-102007 007 198
- US-A- 4 185 629
- US-A1- 2004 184 953
- US-A1- 2013 319 917

## Beschreibung

Die Erfindung betrifft eine extrakorporale Blutbehandlungseinheit, die zum Auswaschen von Gasblasen aus einem Zielbereich eines extrakorporalen Blutkreislaufs ausgelegt ist.

Mikroblasen sind Gasblasen mit einem Durchmesser von wenigen µm, die aufgrund ihrer geringen Größe in der Regel nicht mehr sichtbar sind. Sie entstehen an verschiedenen Stellen und unter verschiedenen Bedingungen in extrakorporalen Blutkreisläufen, unter anderem durch Austreten von blutlöslichen Gasen oder Lufteintritt an kleinsten Undichtigkeiten im Unterdruckbereich des extrakorporalen Kreislaufs, und haften an der inneren Oberfläche des extrakorporalen Schlauchsystems oder der Blutbehandlungseinheit an. Ihre Auswirkungen, wenn sie losgelöst und in den menschlichen Körper infundiert werden, sind stärker als bisher vermutet. Beispielsweise wurden Mikroblasen in lebenswichtigen Organen wie Lunge, Herz und Hirn von Dialysepatienten nachgewiesen.

Beobachtungen an Dialysegeräten mit Hilfe eines Mikroblasendetektors lassen eine Reihe von möglichen Quellen für Mikroblasen erkennen. Unter anderem wurde beobachtet, dass in den ersten Minuten der Behandlung ein erhöhter Eintrag von Mikroblasen in den Patienten stattfindet. Am Anfang kann der mittlere Mikroblasenfluss um eine Größenordnung höher sein als während der restlichen Behandlung. Außerdem gibt es potentiell Luftansammlungen im extrakorporalen Kreislauf, die als Quelle und Reservoir für spätere Mikroblasenbildung dienen können. Luftansammlungen können beispielsweise im Dialysator, im Schlauchsegment der Blutpumpe, in der venösen Tropfkammer, in Heparin- und anderen Injektionsspritzen, in zuführenden Leitungen (z.B. in einem Heparinschlauch) und weiteren Schnittstellen (z.B. Luer-Verbindungen) vorhanden sein. Grundsätzlich begünstigt der Unterdruck im Saugbereich der Pumpe Lufteinträge. Ferner wurde auch während eines Druckhaltetests, bei dem die Dialysierflüssigkeit temporär nicht mehr erneuert wird und das Blut in den Dialysatorkapillaren nicht mehr mit frischer, entgaster Dialysierflüssigkeit umspült wird, eine erhöhte Anzahl an Mikroblasen beobachtet.

Die EP1892000A1 betrifft ein Verfahren zum Vorbereiten des Dialysators eines Dialysegerätes, das einen Blutkreislauf und einen Dialysierflüssigkeitskreislauf aufweist, wobei der Dialysator eine Blutkammer und eine Dialysierflüssigkeitskammer enthält, zwischen denen sich eine semipermeable Membran befindet. Aus der US2013/319917 A1 sind ein Verfahren und eine Vorrichtung zum Primen eines extrakorporalen Blutkreislaufs bekannt. Die EP0543172A2 offenbart eine Antriebsvorrichtung für eine medizinische Pumpe. Die EP2462965A2 betrifft darüber hinaus allgemein ein Verfahren und Vorrichtungen zum Durchführen einer extrakorporalen Blutbehandlung, sowie ein System und Kit mit solchen Vorrichtungen.

Mikroblasen können aufgrund ihrer geringen Größe und ihres geringen Auftriebes nur eingeschränkt in der venösen Kammer abgeschieden und von dem vorgeschriebenen Schutzsystem zur Vermeidung von Luftinfusion nur bedingt erkannt werden.

Der Erfindung liegt die Aufgabe zu Grunde, die Zahl an Mikroblasen in extrakorporalen Blutkreisläufen bzw. die Infusion von Mikroblasen in den Körper des Patienten zu verringern.

Vor diesem Hintergrund schlägt die Erfindung eine extrakorporale Blutbehandlungseinheit gemäß Anspruch 1 vor.

Gemäß dem Stand der Technik läuft die Spülung des extrakorporalen Blutkreislaufs nur bei geringer und konstanter Flussrate bzw. bei konstantem Flüssigkeitsdruck. Dabei werden zwar große Blasen z.B. in der venösen Kammer abgeschieden, die Menge der Mikroblasen jedoch kaum reduziert. Deshalb wird erfindungsgemäß vorgeschlagen, die Blutpumpenrate während der Spülung so zu anzupassen, dass die Mikroblasen besser entfernt werden können.

In einer Ausführungsform handelt es sich bei dem extrakorporalen Blutkreislauf um den einer Dialysemaschine. Der extrakorporale Blutkreislauf einer Dialysemaschine umfasst eine arterielle Leitung, eine Blutpumpe, einen Dialysator und eine venöse Leitung. In der arteriellen Leitung ist ein arterieller Port zum Anschluss an einen Patienten angeordnet. In der venösen Leitung ist ein venöser Port zum Anschluss an den Patienten angeordnet. Ferner ist ein Einsatz des Verfahrens auch in anderen extrakorporalen Behandlungssystemen denkbar, beispielsweise in Ultrafiltrationsgeräten und Herz-Lungen-Maschinen.

In einer Ausführungsform wird die Spülflüssigkeit anhand einer Pumpe durch den Zielbereich gefördert, und die Variation der Flussrate und/oder des Drucks erfolgt durch eine Änderung der Förderrate der Pumpe.

In einer Ausführungsform erfolgt die Änderung der Förderrate durch ein Anschalten und Abschalten der Pumpe oder ein Drosseln und Erhöhen der Pumpengeschwindigkeit. Unter einem Drosseln ist ein Herunterfahren der Pumpe auf eine Förderrate größer 0 zu verstehen, während unter einem Abschalten ein vollständiger Stopp verstanden wird. Entsprechend geht die Erhöhung von einer Förderrate größer 0 aus, während das Anschalten von einer Förderrate gleich 0 ausgeht.

In einer Ausführungsform handelt es sich bei der Pumpe um eine im extrakorporalen Blutkreislauf angeordnete Blutpumpe oder eine außerhalb des extrakorporalen Blutkreislaufs angeordnete Spülpumpe. Beispielsweise kann eine Spülpumpe in einer Zuleitung von Spülflüssigkeit angeordnet sein.

In einer Ausführungsform weist der extrakorporale Blutkreislauf wenigstens eine Klemme oder ein Ventil auf, und die Variation der Flussrate und/oder des Drucks erfolgt durch eine Ansteuerung der Klemme. Der Begriff einer Klemme umfasst vorliegend auch ein Ventil, das den Durchfluss von Spülflüssigkeit in, durch oder aus dem extrakorporalen Blutkreislauf drosseln oder stoppen kann. Der extrakorporale Blutkreislauf kann beispielsweise eine arterielle und/oder eine venöse Klemme aufweisen. Auch ein sich vom arteriellen oder venösen Port unterscheidender Zulauf bzw. Ablauf von Spülflüssigkeit kann eine Klemme aufweisen. Eine Variation des Drucks und/oder der Flussrate kann beispielsweise durch Schließen einer Klemme am Ablauf der Spülflüssigkeit (venöser Port oder separater Ablauf) und ein Stauen von Flüssigkeit sowie anschließendes Öffnen erreicht werden. Ferner kann eine Variation auch durch Schließen einer Klemme am Zulauf der Spülflüssigkeit sowie anschließendes Öffnen und abruptes Einlassen der Spülflüssigkeit erreicht werden.

In einer Ausführungsform wird die Flussrate und/oder der Druck der Spülflüssigkeit in Stößen erhöht und reduziert. Beispielsweise ist eine periodische Erhöhung und Erniedrigung der Flussrate und/oder des Drucks über einen bestimmten Zeitraum möglich. Die Stöße können eine Dauer von beispielsweise 0,1 s bis 5 s haben.

In einer Ausführungsform zeigen die Stöße einen kontinuierlichen oder abrupten Aufbau und einen kontinuierlichen oder abrupten Abfall der Flussrate und/oder des Drucks der Spülflüssigkeit. Beispielsweise kann ein kontinuierlicher Druckaufbau und ein im Wesentlichen abrupter Druckabfall dadurch erreicht werden, dass eine ablaufseitige bzw. venöse Klemme zum Druckaufbau geschlossen und für den anschließenden Druckabfall wieder geöffnet wird. Bei einer ggf. periodischen Änderung der Förderungsleistung der Pumpe kann anhand der Pumpensteuerung bestimmt werden, wie abrupt der Aufbau oder Abfall der Flussrate und/oder des Drucks ausfällt.

In einer Ausführungsform wird die Amplitude der Stöße so gewählt, dass die Flussrate und/oder der Drucks der Spülflüssigkeit zwischen Tälern und Bergen um wenigstens den Faktor 1,3, den Faktor 1,5 oder den Faktor 2 variiert. Durch eine gewisse Mindestgröße der Amplitude der Stöße wird eine gute Wirkung bei der Ablösung von Gasblasen sichergestellt.

In einer Ausführungsform ist die Flussrate der Spülflüssigkeit im extrakorporalen Blutkreislauf während des Spülvorganges wenigstens zeitweise größer als 550 ml/min oder größer 700 ml/min. In vielen Fällen wird während einer extrakorporalen Blutbehandlung ein Fluss von zwischen 200 ml/min und 550 ml/min gewählt, insbesondere dann, wenn Schlauchsysteme mit einem Innendurchmesser von zwischen 3 und 5 mm verwendet werden. Eine höhere Flussrate während des Spülvorganges kann zu einer besseren Ablösung von Gasblasen führen.

In einer Ausführungsform kann das Verfahren im Rahmen des Primingvorganges für den extrakorporalen Blutkreislaufs durchgeführt werden, und beispielsweise den abschließenden Schritt des Primingvorganges darstellen.

In einer Ausführungsform wird das Verfahren während einer Behandlungsunterbrechung, insbesondere während eines Druckhaltetests durchgeführt. Das Verfahren kann manuell oder automatisch ausgelöst werden. Beispiele für Auslösebedingungen umfassen eine periodische Auslösung (z.B. bei Intervallgrößen von 30 Minuten bis 2 Stunden), oder eine Auslösung in Reaktion auf bestimmte Sensormeldungen (z.B. Messwert des Blasendetektors überschreitet einen Grenzwert, wobei dann unterstellt wird, dass die detektierte Blasenzahl und die nicht detektierte Mikroblasenzahl parallel ansteigen) oder Ereignisse (z.B. Beginn eines Druckhaltetests). Die Spülung kann über einen vorbestimmten Zeitraum erfolgen (z.B., mit einer Dauer von zwischen 1 und 15 Minuten), oder auf der Grundlage unterschiedlicher Parameter manuell oder automatisch bestimmt werden. Auch die Kopplung an die Dauer eines anderen Prozesses wie beispielsweise eines Druckhaltetests ist denkbar.

Ebenfalls offenbart ist eine extrakorporale Blutbehandlungseinheit mit einem extrakorporalen Blutkreislauf, einer Pumpe und einer Steuereinheit, wobei die Steuereinheit ausgebildet ist, um das beschriebene Spülverfahren durchzuführen.

In einer Ausführungsform ist die Steuereinheit so ausgebildet, dass sie das Verfahren automatisch im Rahmen des Primings des extrakorporalen Blutkreislaufs auslöst. Beispielsweise kann das Verfahren den abschließenden Primingschritt darstellen.

In einer Ausführungsform ist die Steuereinheit so ausgebildet, dass sie die Behandlung vor Durchführung des erfindungsgemäßen Spülverfahrens unterbricht und/oder dass sie das Verfahren während einer Unterbrechung der Behandlung auslöst und/oder dass sie die Behandlung nach Durchführung des Verfahrens automatisch fortsetzt. Beispielsweise kann die Steuereinheit ausgebildet sein, die Behandlung eigens zur Durchführung des Verfahrens zu unterbrechen. Die Unterbrechung erfolgt beispielsweise periodisch (z.B. bei Intervallgrößen von 30 Minuten bis 2 Stunden) oder in Reaktion auf bestimmte Sensormeldungen, beispielsweise Meldungen eines in der venösen Leitung angeordneten Gasblasensensors. Ferner kann die Steuereinheit so ausgebildet sein, dass das Verfahren dann ausgelöst wird, wenn die Behandlung ohnehin unterbrochen ist. Beispielsweise kann das Verfahren während eines Druckhaltetests ausgelöst werden. Die Dauer des Verfahrens kann beispielsweise der Dauer eines gleichzeitig durchgeführten Druckhaltetests entsprechen (z.B. zwischen 1 und 5 Minuten).

In einer Ausführungsform ist die Steuereinheit so ausgebildet, dass sie die Menge von durch das Verfahren zugeführter Spülflüssigkeit und/oder durch das Verfahren abgeleiteten Blutes bei der Ermittlung behandlungsspezifischer Parameter, insbesondere bei der Ermittlung des Ultrafiltrationsvolumens berücksichtigt.

In einer Ausführungsform weist der extrakorporale Blutkreislauf einen separaten Zulauf und einen separaten Ablauf für Spülflüssigkeit auf, die sich vom arteriellen bzw. venösen Port unterscheiden. Dadurch kann eine höhere Flexibilität mit Blick auf den Einsatzzeitpunkt des erfindungsgemäßen Spülverfahrens erzielt werden, da ein Auswaschen von Gasblasen auch erfolgen kann, während ein Patient an den Kreislauf angeschlossen ist (z.B. während einer Behandlungspause bzw. während eines Druckhaltetests). Der Zulauf ist vorzugsweise in der arteriellen Leitung angeordnet, typischerweise stromaufwärts oder stromabwärts der Blutpumpe. Der Ablauf ist vorzugsweise in der venösen Leitung angeordnet. Bei dieser Ausführungsform wäre im Rahmen des erfindungsgemäßen Verfahrens auch eine Flussumkehr der Spüllösung denkbar, wobei die Spüllösung vorzugsweise gepulst entgegen der Blutstromrichtung bewegt wird.

Die Erfindung betrifft eine extrakorporale Blutbehandlungseinheit mit einem extrakorporalen Blutkreislauf, einer Pumpe und einer Steuereinheit, wobei die Steuereinheit so ausgebildet ist, dass nach einem Stillstand der Pumpe oder nach einem Betrieb der Pumpe mit gedrosselter Geschwindigkeit die Fördergeschwindigkeit für Blut langsam erhöht wird. Die Steuereinheit dieser Blutbehandlungseinheit kann selbstverständlich ferner ausgebildet sein, um das beschriebene Verfahren durchzuführen.

Diese Ausbildung der Steuereinheit erfolgt vor dem Hintergrund, dass beobachtet wurde, dass das rasche Hochfahren der Blutpumpe eine heftige Erhöhung der Mikroblasenanzahl im den Kreislauf verlassenden Blut nach sich ziehen kann. Daher ist es vorteilhaft, dass die Blutpumpe zu Beginn der Behandlung oder nach einem Stopp/einer Reduktion während der Behandlung langsam hochfährt.

Erfindungsgemäß ist die Steuereinheit so ausgebildet, dass die Fördergeschwindigkeit für Blut zu Beginn der Behandlung langsam erhöht wird. Vor dem Beginn der Behandlung steht die Pumpe in der Regel still.

Die Steuereinheit ist so ausgebildet, dass die Fördergeschwindigkeit für Blut nach einer Behandlungsunterbrechung langsam erhöht wird. Während einer Behandlungsunterbrechung kann die Pumpe entweder stillstehen oder mit reduzierter Förderleistung betrieben werden.

Erfindungsgemäß wird die Fördergeschwindigkeit auf einer ggf. linearen Rampe mit einer Steigerung von nicht mehr als 300 ml/min² erhöht. Dies würde bedeuten, dass nach einem Pumpenstopp ein Sollwert für die Behandlung, beispielsweise 400 ml/min, erst nach mindestens einer Minute, beispielsweise erst nach 2 Minuten erreicht wird. Denkbar ist dabei eine stufenweise Erhöhung der Geschwindigkeit, oder eine lineare Erhöhung, z.B. mit 200 ml/min² als Blutbeschleunigung im Anlaufen.

In einer Ausführungsform handelt es sich bei der erfindungsgemäßen extrakorporalen Blutbehandlungseinheit um eine Dialysemaschine. Die Dialysemaschine kann zur Durchführung einer Hämodialyse, einer Hämodiafiltration, einer Hämofiltration und/oder einer reinen Ultrafiltration geeignet und bestimmt sein. Ferner kann es sich bei der erfindungsgemäßen extrakorporalen Blutbehandlungseinheit um ein anderes Behandlungssystemen handeln, beispielsweise ein Ultrafiltrationsgerät oder eine Herz-Lungen-Maschine. Bei der Blutpumpe und/oder der Spülpumpe kann es sich um eine peristaltische Pumpe handeln. Bei der Spülflüssigkeit kann es sich beispielsweise um eine Substitutions- bzw. Priminglösung handeln. Beispiele umfassen physiologische Kochsalzlösung und Ringer-Lösung. Gegebenenfalls kann die Lösung ferner antikoagulative Mittel wie beispielsweise Heparin enthalten.

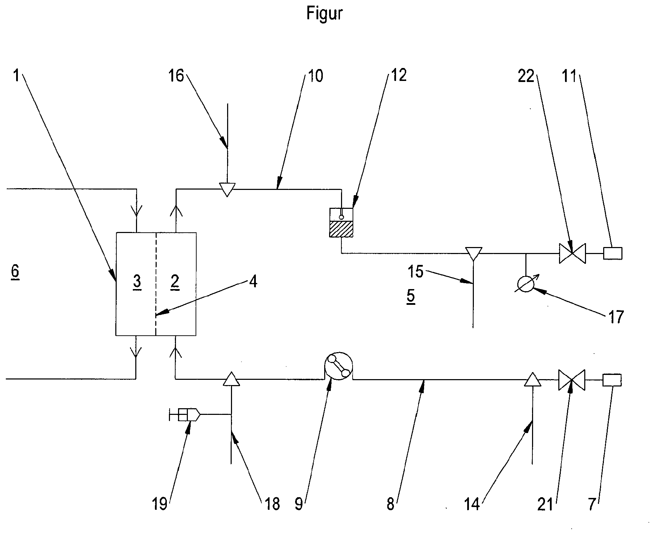

Weitere Einzelheiten und Vorteile ergeben sich aus der nachfolgend beschriebenen Figur und den Ausführungsbeispielen. Die einzige Figur zeigt eine schematische Darstellung von Fluidkreisläufen einer erfindungsgemäßen Dialysemaschine.

Die Dialysemaschine weist einen Dialysator 1 auf, der eine Blutkammer 2 und eine Dialysierflüssigkeitskammer 3 aufweist, die durch eine Membran 4 voneinander getrennt sind. Die Blutkammer 2 ist Bestandteil eines extrakorporalen Blutkreislaufs 5. Die Dialysierflüssigkeitskammer 3 ist. Bestandteil eines Dialysierflüssigkeitskreislaufs 6. Der Blutkreislauf 5 weist einen arteriellen Port 7 auf, der über eine arterielle Leitung 8 mit der Blutkammer 2 verbunden ist. In der arteriellen Leitung 8 sitzt eine Blutpumpe 9. Das venöse Ende der Blutkammer 2 ist anhand der venösen Leitung 10 mit dem venösen Port 11 verbunden. In der venösen Leitung befindet sich eine Tropfkammer 12.

Ferner weist der extrakorporale Blutkreislauf 5 einen separaten Zulauf 14 und einen separaten Ablauf 15 für Spülflüssigkeit auf. Alternativ ist selbstverständlich möglich und von der Erfindung umfasst, dass die Spülflüssigkeit über den arteriellen Port 7 in den extrakorporalen Blutkreislauf eintritt und/oder diesen über den venösen Port 11 wieder verlässt. Auch ein Kurzschluss des arteriellen und venösen Ports zum Zwecke eines Umlaufbetriebes ist denkbar. Gleichwohl kann es vorteilhaft sein, wenn die mit Mikroblasen angereicherte Spülflüssigkeit über den venösen Port oder einen separaten Ablauf aus dem Kreislauf abfließt. Eine Umkehr des Flusses der Blutstromrichtung entgegen der Blutstromrichtung ist denkbar.

Der Ablauf 15 ist in der venösen Leitung 10 stromabwärts aller bestehenden Schnittstellen des extrakorporalen Blutkreislaufs angeordnet. Beispielhaft ist in der Figur als Schnittstelle ein Postdilutions-Port 16 dargestellt. Zwischen Ablauf und venösem Port befindet sich ein Luftblasendetektor 17. Der Zulauf ist in der arteriellen Leitung stromaufwärts der Blutpumpe angeordnet und stellt einen separaten Zugang dar. Als weitere Schnittstelle umfasst der Blutkreislauf einen Prädilutions-Port 18 mit integrierter Heparinzuleitung 19. Der extrakorporale Blutkreislauf umfasst ferner eine arterielle Klemme 20 und eine venöse Klemme 21.

Die arterielle Klemme ist zwischen arteriellem Port 7 und Zulauf 14 angeordnet. Die venöse Klemme ist zwischen Ablauf 15 und venösem Port 11 angeordnet. Sowohl der Zulauf 14 als auch der Ablauf 15 sind anhand eines Dreiwegeventils mit dem extrakorporalen Blutkreislauf 15 verbunden.

Der gezeigte extrakorporale Blutkreislauf kann anhand des beschriebenen Verfahrens gespült werden. So wird am separaten Zulauf 14 Spülflüssigkeit zugeführt und anhand der Blutpumpe 9 durch den Kreislauf gefördert. Während des Spülvorganges wird über einen gewissen Zeitraum, beispielsweise 3 Minuten die Blutpumpe nicht mit konstanter Förderrate betrieben, sondern die Pumpengeschwindigkeit wird in kurzen periodischen Abständen von etwa 2 Sekunden um den Faktor 2 erhöht und wieder erniedrigt. Dies führt zu einer periodischen Änderung der Flussrate und des Drucks der Spülflüssigkeit im extrakorporalen Blutkreislauf, was wiederum die Ablösung von Mikroblasen begünstigt.

Die Spülung kann beispielsweise während des Primings oder während einer Behandlungspause durchgeführt werden. Beispielsweise ist denkbar, das Verfahren während der Dauer eines Druckhaltetests durchzuführen.

Sofern der Prozess während des Primings durchgeführt wird, kann durch Anpassen der Pumpenrate beim initialen Spülen des extrakorporalen Kreislaufs eine Ablösung von bereits vorhandenen Mikroblasen herbeigeführt werden. Der extrakorporale Kreislauf kann maximal mit entgaster Spüllösung befüllt werden. Durch wiederholtes Anhalten und Weiterfahren der Blutpumpe können Druckstöße erzeugt werden. Dadurch und durch eine im weiteren Verlauf erhöhte Pumpenrate können Mikroblasen von der Schlauchwandung abgelöst und durch die Spüllösung abtransportiert werden. Die Spüllösung kann verworfen werden.

Wird nach dem Stillstand der Blutpumpe nach Beendigung des Verfahrens die extrakorporale Blutbehandlung fortgesetzt, so ist im Rahmen des beschriebenen Ausführungsbeispiels vorgesehen, dass die Fördergeschwindigkeit der Blutpumpe zu Beginn der Behandlung langsam erhöht wird. So kann eine Ablösung von Mikroblasen aus dem extrakorporalen Leitungssystem bei Behandlungsbeginn verringert werden. Konkret ist vorgesehen, dass die Fördergeschwindigkeit auf einer linearen Rampe mit einer Steigerung von 200 ml/min² erhöht wird, sodass der Sollwert von 400 ml/min erst nach 2 Minuten erreicht wird. Selbstverständlich sind auch andere Algorithmen für die Erhöhung und andere Sollwerte von der Erfindung umfasst.

Abschließend kann festgehalten werden, dass die teilweise stationären Mikroblasen

im Rahmen des Verfahrens beispielsweise mittels wiederholtem Stop and Go der Blutpumpe und/oder hoher Pumpengeschwindigkeit abgelöst werden, und mittels vorhandener und ggf. zu schaffender Senken im venösen Zweig des extrakorporalen Kreislaufs (z.B. mit der UF-Pumpe über die Dialysatormembran, oder den Substitutionsport oder eine weitere Ableitung im venösen Zweig) aus dem extrakorporalen Kreislauf entfernt werden. Während des initialen Füllens des extrakorporalen Blutkreislaufs mit entgaster Substitutionsflüssigkeit kann der Kreislauf maximal gefüllt werden, und die Luftansammlungen und Mikroblasenreservoire können mittels Fluss- und Druckstößen (z.B. durch kurzzeitige maximale Blutpumpenrate, Sperren und Lösen der venösen Klemme, etc.) angegriffen und reduziert werden. Nach einem Stopp der Blutpumpe vor oder während der Behandlung kann diese stets langsam hochgefahren werden, z.B. in einer Rampe mit nicht mehr als 200 ml/min², was bedeutet, dass nach einem Blutpumpenstopp eine mögliche Sollrate von 400ml/min erst nach 2 Minuten erreicht würde. Im Falle eines Druckhaltetests kann die Blutpumpenrate rechtzeitig vor dem Test abgesenkt werden, die Förderrate von Blutpumpe und Substitutionspumpe während des Bypass minimal bleiben (z.B. Pumpenstopp), und beide Raten nach dem Bypass langsam wieder auf ihren Sollwert hochgefahren werden.

## Patentansprüche

1. Extrakorporale Blutbehandlungseinheit, vorzugsweise Dialysemaschine, mit
einem extrakorporalen Blutkreislauf (5),
einer Pumpe (9), und
einer Steuereinheit,
die dazu ausgelegt ist, während oder am Beginn einer Behandlung eines Patienten mit Hilfe der extrakorporalen Blutbehandlungseinheit, nach einem Stillstand der Pumpe (9) oder nach einem Betrieb der Pumpe (9) mit gedrosselter Geschwindigkeit, die Fördergeschwindigkeit für Blut mittels der Pumpe (9) auf einer Rampe mit einer Steigerung von nicht mehr als 300 ml/min² zu erhöhen, um die Anzahl an Mikroblasen in dem Blut zu minimieren, **dadurch gekennzeichnet, dass** die Steuereinheit ferner dazu ausgelegt ist, ein Spülverfahren zum Auswaschen von Gasblasen aus einem Zielbereich eines extrakorporalen Blutkreislaufs (5) mit einer Spülflüssigkeit durchzuführen, in dem:
die Flussrate der Spülflüssigkeit im extrakorporalen Blutkreislauf (5) während des Spülvorganges außerhalb eines Bereichs möglicher Flussraten liegt, der während der Behandlung für das Blut angewandt wird, nämlich bei größer als 550 ml/min, bevorzugterweise bei größer als 700 ml/min.

2. Einheit nach Anspruch 1, wobei die Fördergeschwindigkeit auf einer Rampe mit einer Steigerung von nicht mehr als 200 ml/min², vorzugsweise 150 ml/min² erhöht wird.

3. Einheit nach Anspruch 2, wobei die Flussrate und/oder der Druck der Spülflüssigkeit im extrakorporalen Blutkreislauf (5) während des Spülvorgangs inkonstant ist.

4. Einheit nach einem der vorhergehenden Ansprüche 2 oder 3, wobei die Steuereinheit ferner dazu ausgelegt ist, die Flussrate und/oder den Druck der Spülflüssigkeit in Stößen zu erhöhen und zu reduzieren.

5. Einheit nach Anspruch 4, wobei die Steuereinheit ferner dazu ausgelegt ist, die Amplitude der Stöße so zu wählen, dass die Flussrate und/oder der Druck der Spülflüssigkeit zwischen Tälern und Bergen um wenigstens den Faktor 1,3, vorzugsweise wenigstens den Faktor 1,5, und bevorzugterweise wenigstens den Faktor 2 variiert.

## Claims

1. An extracorporeal blood treatment unit, preferably a dialysis machine, having an extracorporeal blood circuit (5), a pump (9) and a control unit that is configured to increase the conveying speed for blood by means of the pump (9) on a ramp having an increase of no more than 300 ml/min² during or at the start of a treatment of a patient with the aid of the extracorporeal blood treatment unit after a standstill of the pump (9) or after an operation of the pump (9) at a throttled speed to minimize the number of microbubbles in the blood, **characterized in that** the control unit is further configured to carry out a flushing method for purging gas bubbles from a target region of an extracorporeal blood circuit (5) using a flushing liquid, in which method:
the flow rate of the flushing liquid in the extracorporeal blood circuit (5) is outside a range of possible flow rates that is used during the treatment for the blood, namely at greater than 500 ml/min, preferably at 700 ml/min.

2. A unit in accordance with claim 1, wherein the conveying speed is increased on a ramp having an increase of no more than 200 ml/min², preferably 150 ml/min².

3. A unit in accordance with claim 2, wherein the flow rate and/or the pressure of the flushing liquid in the extracorporeal blood circuit (5) is inconstant during the flushing process.

4. A unit in accordance with one of the preceding claims 2 or 3, wherein the control unit is further configured to increase and to decrease the flow rate and/or the pressure of the flushing liquid in bursts.

5. A unit in accordance with claim 4, wherein the control unit is further configured to select the amplitude of the bursts *such* that the flow rate and/or the pressure of the flushing liquid between valleys and peaks varies by at least the factor 1.3, preferably by at least the factor 1.5 and more preferably by at least the factor 2.

## Revendications

1. Unité de traitement de sang extracorporelle, de préférence machine de dialyse, comprenant
un circuit de sang extracorporel (5),
une pompe (9), et
une unité de commande,
qui est conçue pour, pendant ou au début d'un traitement d'un patient à l'aide de l'unité de traitement de sang extracorporelle, après un arrêt de la pompe (9) ou après un fonctionnement de la pompe (9) à vitesse réduite, augmenter la vitesse de transport pour le sang au moyen de la pompe (9) sur une rampe avec un accroissement ne dépassant pas 300 ml/min², pour minimiser le nombre de microbulles dans le sang, **caractérisée en ce que** l'unité de commande est en outre conçue pour exécuter un procédé de lavage pour éliminer par lavage des bulles de gaz d'au moins une zone cible d'un circuit de sang extracorporel (5) avec un liquide de lavage, dans lequel :
le débit du liquide de lavage dans le circuit de sang extracorporel (5) pendant le processus de lavage est situé en dehors d'une plage de débits possibles, qui est appliquée pour le sang pendant le traitement, à savoir supérieur à 550 ml/min, plus préférentiellement supérieur à 700 ml/min.

2. Unité selon la revendication 1, dans laquelle la vitesse de transport est augmentée sur une rampe avec un accroissement ne dépassant pas 200 ml/min², de préférence 150 ml/min².

3. Unité selon la revendication 2, dans laquelle le débit et/ou la pression du liquide de lavage dans le circuit de sang extracorporel (5) est inconstant(e) pendant le processus de lavage.

4. Unité selon l'une des revendications précédentes 2 ou 3, dans laquelle l'unité de commande est en outre conçue pour augmenter et réduire le débit et/ou la pression du liquide de lavage par à-coups.

5. Unité selon la revendication 4, dans laquelle l'unité de commande est en outre conçue pour sélectionner l'amplitude des à-coups de telle manière que le débit et/ou la pression du liquide de lavage varie entre les creux et les pics d'au moins un facteur 1,3, de préférence au moins le facteur 1,5, et plus préférentiellement au moins le facteur 2.
